Europäisches Patentamt
European Patent Office
Office européen des brevets

(19) (11) Publication number: **0 038 629**
**A1**

(12) # EUROPEAN PATENT APPLICATION

(21) Application number: 81301242.4

(22) Date of filing: 23.03.81

(51) Int. Cl.³: C 07 H 19/04
A 61 K 31/70, C 07 C 87/47

(30) Priority: 17.04.80 GB 8012621

(43) Date of publication of application:
28.10.81 Bulletin 81/43

(84) Designated Contracting States:
CH DE FR GB IT LI

(71) Applicant: BEECHAM GROUP LIMITED
Beecham House Great West Road
Brentford, Middlesex(GB)

(72) Inventor: Harnden, Michael Raymond
47 Guildford Road
Horsham Sussex(GB)

(72) Inventor: Bailey, Stuart
14 Overdale
Dorking Surrey(GB)

(74) Representative: Russell, Brian John et al,
European Patent Attorney Beecham Pharmaceuticals
Yew Tree Bottom Road
Epsom Surrey, KT18 5XQ(GB)

(54) **Virazole derivatives, process for their preparation and use as antiviral agents.**

(57) A compound of the formula (II)

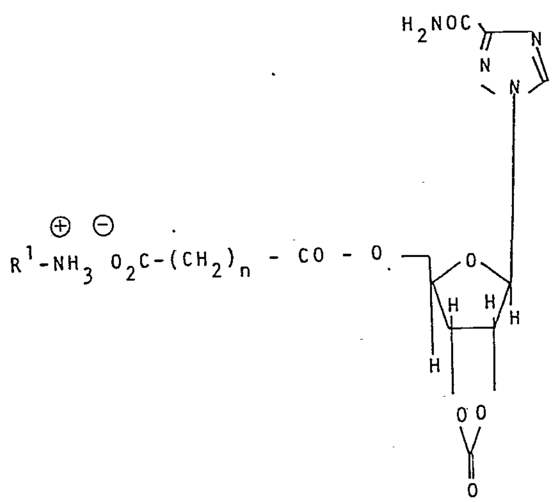

(11)

in which $R^1$ is a group such that $R^1NH_2$ is an antiviral lipophilic amine and n is 2 or 3, has antiviral activity, particularly agains influenza A strains.

EP 0 038 629 A1

# VIRAZOLE DERIVATIVES, PROCESS FOR THEIR PREPARATION AND THEIR USE AS ANTIVIRAL AGENTS

This invention relates to virazole derivatives, a process for their preparation and their use as antiviral agents.

1-β-Ribofuranosyl-1,2,4-triazole-3-carboxamide (hereinafter referred to as virazole) is a known antiviral agent (see, for example, British Patent Specification No. 1,353,565 and Streeter et al., Proc. Nat. Acad. Sci. U.S.A., 70, 1174 (1973) ). Virazole 5'-alkyl esters are also known to share this activity as is virazole 2',3',5'-triacetate and virazole phosphates (see, for example, Sidwell et al., J. Med. Chem., 21, 742 (1978)). It is also known that certain lipophilic amines such as cyclooctylamine, 1-aminoadamantane, 1-(adamant-1-yl)-1-aminoethane and spiro-amantadine possess antiviral activity. It has also been reported that mixtures of virazole and 1-(adamant-1-yl)-1-aminoethane display good *in vitro* activity against influenza virus (see, for example, Galegov et al., Experientia, 33, 905, (1977) ). One disadvantage with virazole is that it is a metabolic inhibitor which inhibits weight gain in animals. It would clearly be desirable to find antiviral agents which do not suffer from this disadvantage. Such a class of agents has now been discovered.

According to the present invention there is provided a compound of the formula (I):

(I)

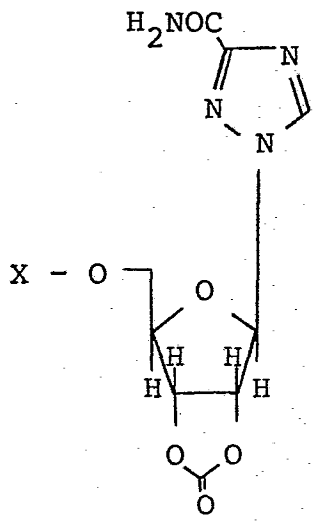

or a salt thereof, wherein X is a hydrogen atom or a $HO_2C(CH_2)_nCO$ group, and n is 2 or 3.

Preferred compounds according to the invention are amine salts of formula (II):

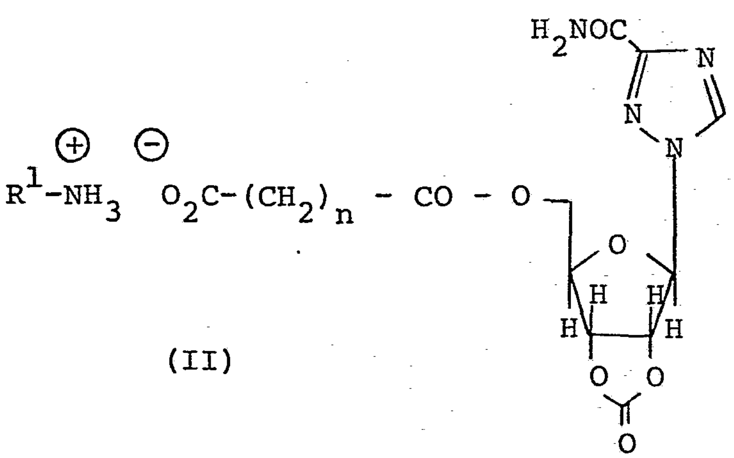

(II)

wherein $R^1$ is a group such that $R^1NH_2$ is an antiviral lipophilic amine, and n is as defined in formula (I).

Suitable antiviral lipophilic amines are alkyl primary amines containing from 8 to 14 carbon atoms and at least one ring. Such amines include amantadine, rimantadine, spiroamantadine and cyclooctylamine. The preferred antiviral lipophilic amine is amantadine.

Other suitable salts of the compounds of formula (I) are alkali or alkaline earth metal salts or salts with anion exchange resins.

In order to use the compounds of the invention as antiviral agents they are formulated in pharmaceutical compositions for human or veterinary use by standard methods in several dosage forms.

Accordingly, the invention provides a pharmaceutical composition comprising a compound of the formula (I), or a salt thereof, together with a pharmaceutically acceptable carrier.

Compounds which are active when given by the oral route may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The salts

may also be presented with a sterile liquid carrier for injection.

In man, influenza virus typically infects the upper respiratory tract and may be effectively treated by topical application of, for example, nose drops. Alternatively, the compositions of this invention may be presented as a microfine powder for insufflation (in such a case the particles of active compound suitably have diameters of less than 50 microns, preferably less than 10 microns) or in the form of an aerosol or a solution for a nebuliser.

Preferably the compositions of this invention are in unit dosage form or in some other form that the patient may administer to himself a single dose. A suitable dosage unit might contain from 1 to 250 mg of active ingredient, for example 2 to 100 mg. Such doses may be administered 1 to 6 times a day or more usually 3 to 4 times a day. The effective dose of salt depends on the particular salt employed, but is in general in the range of from 0.1 mg/kg/day to 20 mg/kg of body weight per day or more usually 0.5 mg/kg/day to 10 mg/kg/day.

The compounds of formula (I) in which X is a hydrogen atom may be prepared by treating virazole with a tritylating agent followed by a carbonating agent and subsequently removing the trityl group. A preferred tritylating agent is trityl chloride and a preferred carbonating agent is 1, 1'-carbonyl-diimidazole.

The salts of the formula (II) may be prepared by reacting the chosen amine $R^1NH_2$ with a compound of the formula (III):

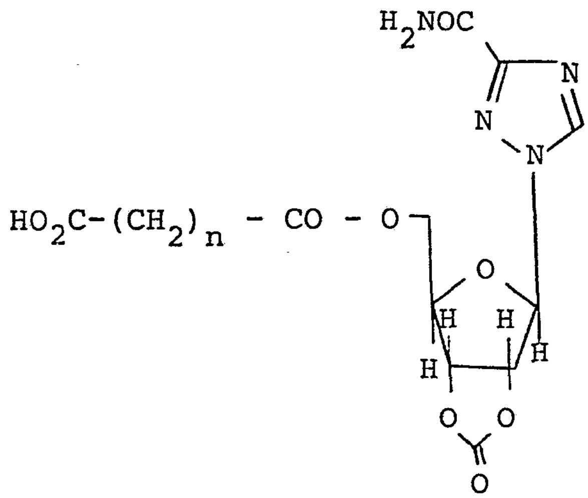

(III)

Accordingly in a further aspect the invention provides a process for preparing a salt of the formula (II) as hereinbefore defined which process comprises reacting an amine $R^1NH_2$ where $R^1$ is as hereinbefore defined with a compound of the formula (III) as hereinbefore defined.

The reaction is conveniently carried out by mixing a solution of the amine with a solution of the compound of the formula (III) and thereafter where desired removing the solvent. The reaction is best carried out at moderate temperatures. The precise temperature is not critical, but should be selected so that the reagents at least are not deposited from solution. We have found room temperature to be convenient. Suitable solvents for the above process may be determined by trial and error. Generally, the solvents are polar organic solvents such as lower alkanols or aqueous mixures thereof or water adjusted where necessary to pH at which the reagents are soluble. A preferred solvent is methanol optionally together with water.

The compounds of formula (III) may themselves be prepared by treating a compound of formula (I) in which X is a hydrogen atom with an acid anhydride of formula

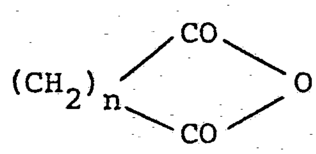

in which n is 2 or 3. The reaction is preferably carried out in the presence of an acid acceptor, such as 4-dimethylaminopyridine, in a polar solvent, such as pyridine.

The compounds of the formulae (I), (II) and (III) may be prepared as shown in the following scheme:

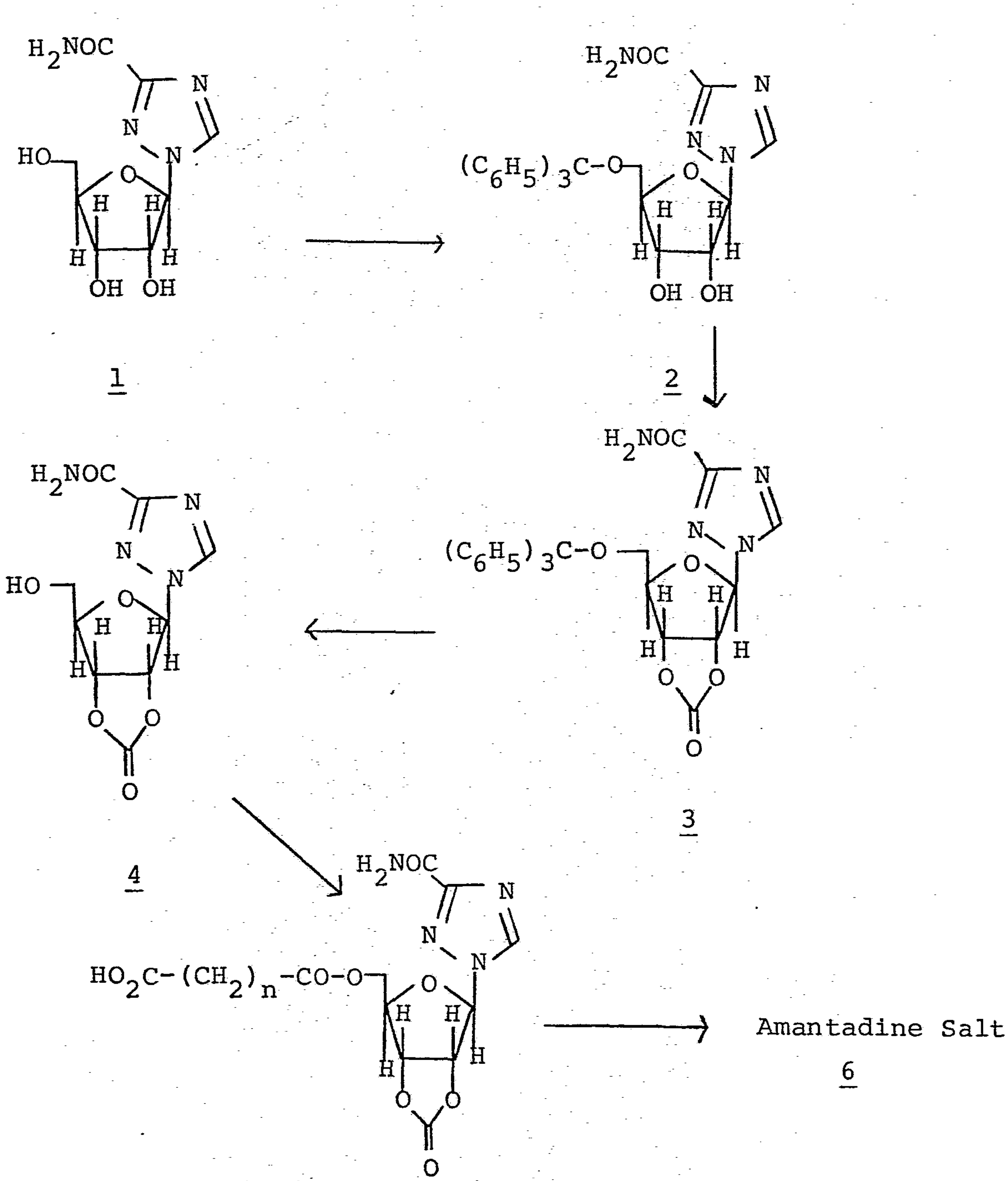


1 2 3 4 5 6

The esterification of 1-[β-D-(2', 3'-carbonyl)ribofuranosyl]-3-carbamoyl-1,2,4-triazole may be effected by the reaction with succinic anhydride in the presence of an acid acceptor such as 4-dimethylaminopyridine in a solvent such as pyridine. Such reactions are normally carried out at an elevated temperature such as 100°C. Evaporation of the solvent yields the initial crude product which may then be purified in conventional manner such as by chromatography. The acid-ester may be neutralised in conventional manner to provide salts if these are required.

The virazole carbonate may be prepared as described in the Examples.

The following Examples illustrate the invention:

Example 1

1-[β-D-(5'-Trityl)ribofuranosyl]-3-carbamoyl-1,2,4-triazole (2)

A solution of 1-β-D-ribofuranosyl-3-carbamoyl-1,2,4-triazole (1) (10 g, 0.041 mol) and trityl chloride (12,4 g, 0.045 mol) in pyridine (120 ml) was stirred at room temperature for 18 hours and then heated on a steam bath for 0.5 hour. The pyridine was removed under reduced pressure to leave a yellow oil. A t.l.c. of this oil on silica eluted with 9:1 acetonitrile-0.1M ammonium chloride showed trityl alcohol moving with the solvent front, a major component at $R_f$ 0.6 with unreacted virazole at $R_f$ 0.25. The oil was dissolved in hot ethanol and refrigerated to yield about 5 g of tritylated virazole. The ethanol was removed from the liquors and the residue dissolved in 5:1 ethyl acetate - methanol. The solution was chromatographed on silica eluting with 10:1 ethyl acetate - methanol to obtain 1-[β-D-(5'-trityl)ribofuranosyl]-3-carbamoyl-1,2,4-triazole (2) as a white solid in a total yield of 73% (14.5 g), m.p. 209-212°C; $\nu_{max}$ (nujol) 3400 ($NH_2$, OH), 1710, 1650, 1600, 1590 $cm^{-1}$; $^1H$ n.m.r. [$(CD_3)_2SO$] δ3.0-3.3 (2H, m, 5'-$CH_2$), 3.9-4.2 (1H, m, 4'-CH), 4.32 (1H, m, 3'CH), 4.4 (1H, m, 2'CH) 5.2 (1H, br.m, $D_2O$ exchangeable, OH), 5.67 (1H, br.m, $D_2O$ exchangeable, OH), 5.95 (1H, d, J = 2, 1'CH), 6.9-7.5 (15H, br.m, aromatic), 7.5-7.8 (2H, br.m, $D_2O$ exchangeable, $NH_2$), 8.82 (1H, s, triazole CH). Found: C, 66.57; H, 5.33; N, 11.50%. Required for $C_{27}H_{26}N_4O_5$: C, 66.65; H, 5.39; N, 11.52%.

Example 2

1-[β-D-(5'-Trityl-2',3'-carbonyl)ribofuranosyl]-3-carbamoyl-1,2,4-triazole (3)

A solution of 1-[β-D-(5'-trityl)ribofuranosyl]-3-carbamoyl-1,2,4-triazole (2) (5.6 g, 0.0116 mol) in pyridine (50 ml) was stirred and treated with 1,1'-carbonyldiimidazole (2.2 g, 0.0136 mol). After 1 hour at room temperature t.l.c. in acetonitrile showed one major product ($R_f$ 0.53). The pyridine was removed under reduced pressure and the resulting gum dissolved in a mixture of ethyl acetate and chloroform. This solution was washed with water, dried and decolourised with charcoal. The solution was filtered and the solvents removed under reduced pressure to give a white solid which was recrystallised from hot ethyl acetate yielding 5.6 g (96%) of 1-[β-D-(5'-trityl-2',3'-carbonyl)ribofuranosyl]-3-carbamoyl-1,2,4-triazole (3) as a white crystalline solid, m.p. 179-181°C, $\nu_{max}$ (nujol) 3400 ($NH_2$), 1800, 1715, 1700, 1600 $cm^{-1}$; $^1H$ n.m.r. [$(CD_3)_2SO$] δ 3.15 (2H, d, J = 6, 5'$CH_2$), 4.65 (1H, m, 4'CH), 5.65 (2H, m, 2'CH, 3'CH), 6.65 (1H, s, 1'CH), 7.3 (15H, m, aromatic), 7.65 (2H, br.s, slow $D_2O$ exchangeable, $NH_2$), 8.85 (1H, s, triazole CH). Found: C, 64.84; H, 4.85; N, 10.66% Required for $C_{28}H_{24}N_4O_6$: C, 65.62; H, 4.72; N, 10.93%.

Example 3

1-[β-D-(2',3'-carbonyl)ribofuranosyl]-3-carbamoyl-1,2,4-triazole (4)

A solution of 1-[β-D-(5'-Trityl-2',3'-carbonyl) ribofuranosyl]-3-carbamoyl-1,2,4-triazole (3) (8.0 g, 0.0156 mol) in a mixture of n-butanol/trifluoroacetic acid (3:1) (800 ml) was stirred at room temperature for 1 hour. More n-butanol (400 ml) was added and the solution concentrated to low volume until crystallisation started. The mixture was refrigerated, the white solid filtered off and washed with ethanol to give 3.0 g (71%) of 1-[β-D-(2',3'-carbonyl)ribofuranosyl]-3-carbamoyl-1,2,4-triazole (4). m.p. 207-209°C; $\nu_{max}$ (nujol) 3200-3500 ($NH_2$, NH,CH) 1800, 1700, 1670, 1620 $cm^{-1}$; $^1H$ n.m.r. [$(CD_3)_2SO$] δ 3.5 (2H, d, J = 6, 5'$CH_2$), 4.5 (1H, dt, J = 2, 4'CH), 5.15 (1H, t, J = 5, $D_2O$ exchangeable, OH), 5.47 (1H, dd, J = 2, J = 6, 3'CH), 5.82 (1H, d, J = 6, 2'CH), 6.6 (1H, s, 1'CH, 7.0 (1H, br.s, slow $D_2O$ exchangeable, NH), 7.52 (1H, br.s, slow $D_2O$ exchangeable, NH),8.85 (1H, s, triazole CH). Found: C, 40.16; triazole H, 3.90; N, 20.59%. Required for $C_9H_{10}N_4O_6$: C, 40.01; H, 3.73; N, 20.74%.

Example 4

1-[β-D-(5'-Succinyl-2',3'-carbonyl(ribofuranosyl]-3-carbamoyl-1,2,4-triazole (5)

A mixture of 1-[β-D-(2',3'-carbonyl(ribofuranosyl]-3-carbamoyl-1,2,4-triazole (4) (1 g, 3.7 mmol), succinic anhydride (0.45 g, 4.5 mmol) and 4-dimethylaminopyridine (20 mg) was stirred together in pyridine (20 ml) at 100°C for 18 hours. T.l.c. on silica eluting with ethylacetate - methanol 5:1, showed that reaction was complete and the pyridine was removed under reduced pressure. The residue was chromatographed on silica eluting with ethylacetate - methanol 5:1. The solvents were removed from the appropriate fractions and 0.85 g (62%) of 1-[β-D-(5'-succinyl-2',3'-carbonyl)ribofuranosyl]-3-carbamoyl-1,2,4,-triazole (5) was isolated as a white solid; $\nu_{max}$ (KBr) 3400 ($NH_2$), 2550 (COOH), 1810, 1730, 1680, 1600 $cm^{-1}$; $^1H$ n.m.r. [$(CD_3)_2SO$] δ 2.57 (4H, s, 2 x $CH_2$), 4.31 (2H, dd, J = 5.2, J = 7, 5'$CH_2$), 4.8 (1H, m, 4'CH), 5.74 (1H, dd, J = 6.5, J = 2, 3'CH), 6.0 (1H, dd, J = 6.5, J = 0.5, 2'CH), 6.67 (1H, d, J = 0.5, 1'CH), 7.07 (1H, br.s, slow $D_2O$ exchangeable, NH), 7.58 (1H, br.s, slow $D_2O$ exchangeable, NH), 8.82 (1H, s, triazole CH). Found: C, 41.78; H, 4.21; N, 14.76%. Required for $C_{13}H_{14}N_4O_9$: C, 42.17; H, 3.81; N, 15.13%.

Example 5

1-[β-D-(5'-succinyl-2',3'-carbonyl)ribofuranosyl]-3-carbamoyl-1,2,4-triazole amantadine salt (6)

A solution of 1-[β-D-(5'-succinyl-2',3'carbonyl) ribofuranosyl]-3-carbamoyl-1,2,4-triazole (5) (0.25 g, 0.68 mmol) in water-methanol (10 ml) was stirred during the addition of amantadine (0.102 g, 0.68 mmol) in methanol (5 ml). The pH of the solution was found to be pH 6.3, and the solvents were removed under reduced pressure. The residual white foam was triturated with acetone and 0.26 g (74%) of 1-[β-D-(5'-succinyl-2',3'-carbonyl)ribofuranosyl]-3-carbamoyl-1,2,4-triazole amantadine salt (6) obtained as a white solid, m.p. 126 - 128°C, $\nu_{max}$ (KBr) δ 3400-3100, 1810, 1730, 1685, 1560 $cm^{-1}$; $^1H$ n.m.r. [$(CD_3)_2SO$] δ1.4-2.6 (19H, m, 2 x $CH_2$ + amantadine Hs), 4.15 (2H, m, 5'$CH_2$), 4.7 (1H, t, J = 6, 4'CH), 5.6 (1H, dd, J = 2, J = 6, 3'CH), 5.85 (3H, br.s, $D_2O$ exchangeable, $-\overset{+}{N}H_3$), 5.85 (1H, d, J = 6, 2'CH), 6.61 (1H, s, 1'CH), 7.65 (1H, br.s, slow $D_2O$ exchangeable, NH), 7.88 (1H, br.s, slow $D_2O$ exchangeable, NH), 8.86 (1H, s, triazole CH). Found: C, 51.61; H, 6.12; N, 12.84%. Required for $C_{23}H_{31}N_5O_9.H_2O$: C, 51.20; H, 6.16; N, 12.98%.

Demonstration of Effectiveness

Antiviral Activity

In Vitro

Diffusion Test. 10 mg/ml solutions of the compounds in water were diluted to a concentration of 1 mg/ml with Eagle's minimum essential medium and 0.02 ml introduced into wells 6mm in diameter cut into an agar layer above influenza A/Port Chalmers/1/73 (H3N2) virus infected MDCK cell monolayers. An infection level giving an even distribution of subconfluent plaques was used. The diameters of zones around the wells free from virus induced plaques (inhibition zones) and diameters of zones in which cytotoxicity was apparent are reported.

Results.

| Compound | Active Zone (mm)/Toxic Zone (mm) |
|---|---|
| 1 | 28/0 |
| 5 | 17/0 |
| 6 | 55/0 |

Plaque Reduction Assay.

More accurate quantitation of antiviral activity was achieved by incorporated of concentrations of the test compound varying from 1.3 x $10^{-6}$M to 4.1 x $10^{-4}$M into the agar overlay above the influenza A/Port Chalmers/1/73 (H3N2) virus infected MDCK cell monolayers. The % reduction in the number of virus induced plaques at each compound concentration is given below.

| | % Plaque Reduction at Concentration Given | | | | | |
|---|---|---|---|---|---|---|
| | Molarity x $10^{-5}$ | | | | | |
| | 41 | 13 | 4.1 | 1.3 | 0.41 | 0.13 |
| 1 | 100 | 100 | 100 | 94 | 43 | 0 |
| 5 | 0* | 0* | 0* | 0 | 0 | 0 |
| 6 | 100 | 100 | 100 | 89 | 63 | 44 |

* Significant reduction in plaque size, but not in number

In mice

In mice, influenza infection causes inflammation and consolidation of the lung tissue and an increase in the lung weight/body weight ratio. The antiviral activity of a compound can be assessed from its ability to inhibit these influenza-induced effects.

Procedure.

Groups of 10 male Charles River mice of the outbred CD-1 strain were used. The mice weighed about 20g at the start of the experiment and group weights were matched. Infection was by aerosol. Primary stocks of a mouse adapted strain of the $A_2$/HK/1/68 virus were passaged in eggs to

provide a working stock which was stored at -70$^{O}$C. Working dilutions were made in 1:1 nutrient broth: phosphate buffered saline. Virus was nebulized in an airflow of 25 l/min. into a chamber of volume 120 litres. Compounds were administered twice daily for 3 days, the first dose being given 3 hours before infection and the second 3 hours after infection. Infection was with 1/30 $LD_{50}$ of virus administered over 15 minutes. Body weight changes were recorded over the dosing period and used as an indication of drug toxicity. On day +8 post infection the animals were killed with an intraperitoneal injection of barbiturate, the body weights recorded, and then the lungs carefully excisted and weighed. Results were analysed by a method of least significant differences.

Results

| Expt. No. | Compound[a] | Weight Change Day 0 -Day 3 | Weight Change Day 3 -Day 8 | Day 8 Means Lung Wt. (mg) | Day 8 Means Body Wt. (gm) | Day 8 Means Lung WT./ Body Wt. Ratio x 100 |
|---|---|---|---|---|---|---|
| 1 | Uninfected Controls | +2.5 | +3.4 | 207 | 25.2 | 0.83** |
| | 1 | +0.7* | +0.8 | 238 | 20.7 | 1.16 |
| | 6 | +2.4 | +0.9 | 273 | 22.4 | 1.25 |
| | Infected Controls | +2.4 | -1.7 | 284 | 19.9 | 1.46 |
| 2 | Uninfected Controls | +2.8 | +4.7 | 189 | 26.2 | 0.72** |
| | 1 | +0.2* | +1.8 | 291 | 20.5 | 1.36 |
| | 6 | +2.1 | +0.4 | 266 | 21.2 | 1.30** |
| | Infected Controls | +3.4 | -2.6 | 326 | 19.3 | 1.72 |
| 3 | Uninfected Controls | +2.6 | +3.7 | 182 | 25.3 | 0.72** |
| | 1 | +0.9* | -0.8 | 285 | 19.1 | 1.40** |
| | 6 | +2.6 | +0.7 | 275 | 22.5 | 1.25** |
| | Infected Controls | +3.6 | -3.9 | 339 | 18.8 | 1.75 |

a. Each compound tested at $10^{-4}$ mole/kg doses

* Significantly lower than for untreated controls (toxic) ($p<0.05$).

** Significantly lower than for infected untreated controls ($p<0.05$)

In Experiments 1 and 2, the compounds were administered subcutaneously

In Experiment 3, the compounds were administered orally.

CLAIMS

1. A compound of the formula (I):

(I)

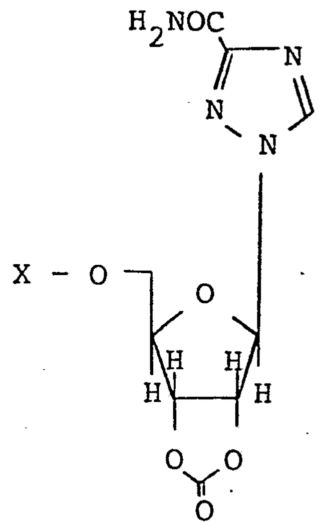

or a salt thereof, wherein X is a hydrogen atom or a $HO_2C(CH_2)_nCO$ group, and n is 2 or 3.

2. A compound according to claim 1, of the formula (II):

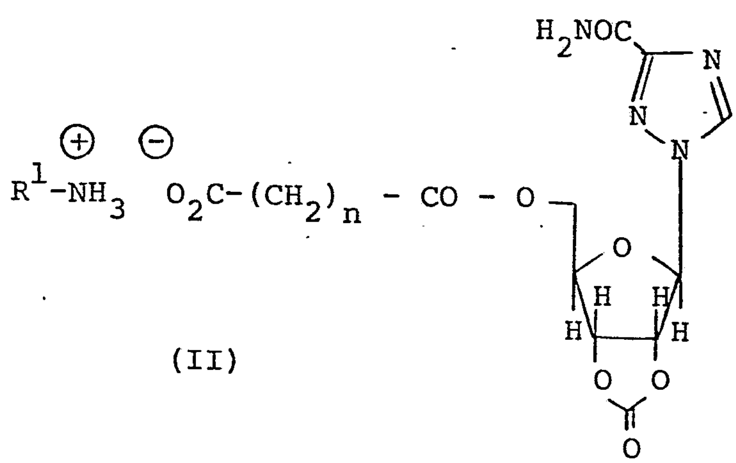

(II)

wherein $R^1$ is a group such that $R^1NH_2$ is an antiviral lipophilic amine, and n is as defined in formula (I).

3. A compound according to claim 2, characterised in that the antiviral lipophilic amine is an alkyl primary amine containing 8 to 14 carbon atoms and at least one ring.

4. A compound according to claim 3, characterised in that the amine is amantadine, rimantadine, spiroamantadine or cyclooctylamine.

5. A pharmaceutical composition comprising a compound of the formula (I), or a salt thereof, together with a pharmaceutically acceptable carrier.

6. A process for preparing a compound of formula (I), in which X is a hydrogen atom, which comprises treating virazole with a tritylating agent, followed by a carbonating agent, and subsequently removing the trityl group.

7. A process for preparing a compound of formula (II):

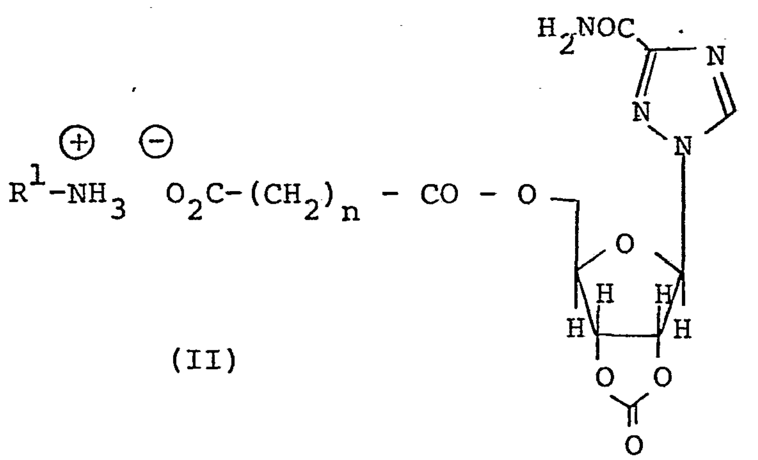

(II)

in which $R^1$ and n are as defined in claim 2, which comprises reacting an amine of formula $R^1NH_2$ with a compound of formula (III).

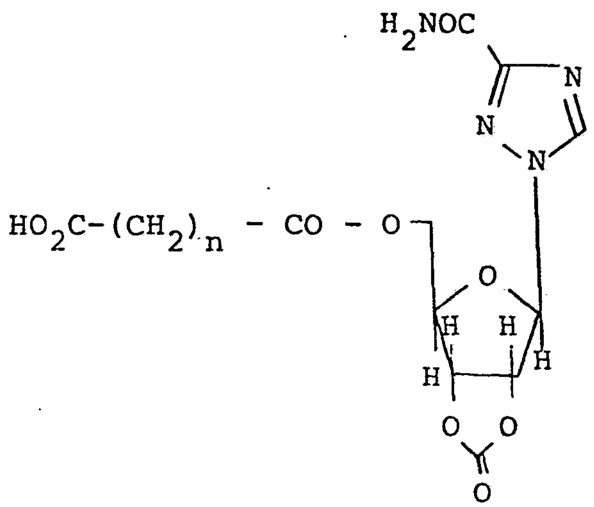

(III)

8. A process for preparing a compound of formula (III) which comprises treating a compound of formula (I), in which X is a hydrogen agom, with an acid anhydride of formula

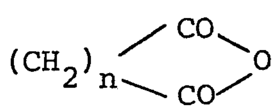

in which n is 2 or 3.

9. A compound according to any one of claims 1 to 4, for use as an antiviral agent.

0038629

European Patent Office

**EUROPEAN SEARCH REPORT**

Application number
EP 81 30 1242

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| D | JOURNAL OF MEDICINAL CHEMISTRY, vol. 21, no. 8, August 1978 pages 742-746 L.B. ALLEN et al.: "Synthesis and Antiviral Activity of Some Phosphates of the Broad-Spectrum Antiviral Nucleoside, 1-β-D-Ribofuranosyl-1,2,4-triazole-3-carboxamide (Ribavirin)" * Page 742 * -- | 1,5 | C 07 H 19/04<br>A 61 K 31/70<br>C 07 C 87/47 |
| | EXPERIENTIA 1977, vol. 33, no. 7 pages 905-906 G.A. GALEGOV et al.: "Combined action of ribovirin and rimantadine in experimental myxovirus infection" * Page 905 * ---- | 1,2,4,5 | TECHNICAL FIELDS SEARCHED (Int. Cl.³)<br>C 07 H 19/04<br>A 61 K 31/70 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 12-06-1981 | VERHULST |

EPO Form 1503.1 06.78